# EUROPEAN PATENT APPLICATION

(11) **EP 3 184 018 A1**
(43) Date of publication of application: **28.06.2017**
(21) Application number: 15833442.5
(22) Date of filing: 27.04.2015
(51) Int. Cl.: A61B 1/00

(54) **GUIDANCE DEVICE AND CAPSULE MEDICAL DEVICE GUIDANCE SYSTEM**

(30) Priority: 21.08.2014 JP 2014168841
(71) Applicant: Olympus Corporation, Tokyo 192-8507 (JP)
(72) Inventor: KAWANO, Hironao, Hachioji-shi Tokyo 192-8507 (JP)
(74) Representative: Schicker, Silvia
(86) International application number: PCT/JP2015/062660
(87) International publication number: WO 2016/027519

(57) **Abstract**

A guidance device and the like are provided which can stably guide and immobilize a capsule medical device in a liquid regardless of individual differences between capsule medical devices. A guidance device 20 includes a magnetic field generation unit 25 that generates a magnetic field to be applied to a permanent magnet 18 provided in a capsule endoscope 10 and a guidance controller 261 that controls the magnetic field generated by the magnetic field generation unit 25. The guidance controller 261 switches between: a first control mode that causes the magnetic field generation unit 25 to generate a magnetic field that guides the capsule endoscope 10 in a direction in which the capsule endoscope 10 moves and comes into contact with an upper or a lower boundary surface of the liquid; a second control mode that continuously changes the magnetic field generated by the magnetic field generation unit 25 so that the capsule endoscope 10 shifts from a state in which the capsule endoscope 10 is in contact with the boundary surface to a state in which the capsule endoscope 10 is not in contact with the boundary surface; and a third control mode that controls the magnetic field generated by the magnetic field generation unit 25 so that the capsule endoscope 10 is held in the liquid.

## Description

### Field

The present invention relates to a guidance device and a capsule medical device guidance system for guiding a capsule medical device introduced into a subject. Background

A capsule medical device has been developed that is introduced into a subject and acquires various kinds of information related to inside of the subject or administers medicine and the like inside the subject. As an example, in the field of endoscope, a capsule endoscope is known that is formed into a size that can be introduced into a digestive tract (a lumen) of the subject.

The capsule endoscope is a device where an imaging function and a wireless communication function are included inside a capsule-shaped casing. After being swallowed by the subject, the capsule endoscope captures images while being moved inside the digestive tract by peristaltic movement and sequentially wirelessly transmits image data of images inside an organ of the subject (hereinafter also referred to as in-vivo images). The wirelessly transmitted image data is received by a receiving device provided outside the subject. Further, the image data is introduced into an image display device such as a workstation and predetermined image processing performed on the image data. As a result, a still image or a moving image of the in-vivo image of the subject can be displayed on a screen of the image display device.

In recent years, a guidance system has been proposed which includes a guidance device that guides a capsule endoscope, which is introduced into a subject, by using a magnetic field (for example, see Patent Literature 1). Generally, in such a guidance system, a permanent magnet is provided inside the capsule endoscope and a magnetic field generation unit such as an electromagnet or a permanent magnet is provided in the guidance device. A liquid such as water is introduced into a digestive tract such as a stomach of the subject and the capsule endoscope is caused to float in the liquid. In this state, the capsule endoscope inside the subject is guided by a magnetic field generated by the magnetic field generation unit. A display unit that receives the image data acquired by the capsule endoscope and displays the in-vivo image is provided to the guidance system, so that a user can operate the guidance of the capsule endoscope by using an operation input unit provided to the guidance device while referring to the in-vivo image displayed on the display unit.

### Citation List

### Patent Literature

Patent Literature 1: Japanese Translation of PCT International Application Publication No. JP-T-2008-503310 Summary

### Technical Problem

When guiding a capsule medical device in a liquid, the position and posture of the capsule medical device are stabilized by causing the capsule medical device to once come into contact with a boundary surface of the liquid, that is, a liquid surface or a liquid bottom surface, and thereafter the capsule medical device is guided to be away from the boundary surface and is immobilized in the liquid. Then, a guidance operation desired by a user is performed. In this case, to immobilize the capsule medical device in the liquid, it is necessary to perform control for generating a magnetic field to guide the capsule medical device under a condition that a gravitational force, a buoyant force, and a magnetic force applied to the capsule medical device are balanced.

However, there are individual differences in masses and volumes of individual capsule medical devices and in sizes of permanent magnets provided inside the individual capsule medical devices. Therefore, it is not possible to stably guide the capsule medical device when performing control by using a uniform strength of magnetic field and a uniform rate of change for changing the magnetic field.

The present invention has been made in view of the foregoing, and an object of the present invention is to provide a guidance device and a capsule medical device guidance system which can stably guide a capsule medical device in a liquid regardless of individual differences between capsule medical devices.

### Solution to Problem

In order to solve the above described problem and achieve the object, a guidance device according to the invention is a guidance device for introducing a capsule medical device having therein a magnet, into a subject in which a liquid is introduced, and guiding the capsule medical device in the liquid by using a magnetic field. The guidance device includes: a magnetic field generation unit configured to generate a magnetic field to be applied to the magnet; and a guidance controller configured to control the magnetic field generated by the magnetic field generation unit. The guidance controller is configured to switch between: a first control mode that causes the magnetic field generation unit to generate a magnetic field that guides the capsule medical device in a direction in which the capsule medical device moves and comes into contact with an upper or a lower boundary surface of the liquid; a second control mode that continuously changes the magnetic field generated by the magnetic field generation unit so that the capsule medical device shifts from a state in which the capsule medical device is in contact with the boundary surface to a state in which the capsule medical device is not in contact with the boundary surface; and a third control mode that controls the magnetic field generated by the magnetic field generation unit so that the capsule medical device is held in the liquid.

The guidance device further includes a determination unit configured to determine whether or not the capsule medical device is in contact with the boundary surface. The guidance controller is configured to switch to the third control mode based on a determination result where the determination unit determines that the capsule medical device is not in contact with the boundary surface in the second control mode.

In the guidance device, based on a control condition for the magnetic field generation unit in switching from the second control mode to the third control mode based on the determination result by the determination unit, the guidance controller is configured to determine a switching condition for subsequent switching from the second control mode to the third control mode.

The guidance device further includes a determination unit configured to determine whether or not the capsule medical device is in contact with the boundary surface. The guidance controller is configured to: further perform a fourth control mode that continuously changes the magnetic field generated by the magnetic field generation unit so that the capsule medical device shifts from a state in which the capsule medical device is in contact with the boundary surface to a state in which the capsule medical device is not in contact with the boundary surface, and acquires a control condition for the magnetic field generation unit at a time when the determination unit determines that the capsule medical device shifts from a state in which the capsule medical device is in contact with the boundary surface to a state in which the capsule medical device is not in contact with the boundary surface; and determine, in the second control mode, a switching condition in switching from the second control mode to the third control mode, based on the control condition acquired by the fourth control mode.

In the guidance device, in switching from the second control mode to the third control mode, the guidance controller is configured to control the magnetic field generation unit such that an amount of change in a magnetic field per unit time when continuously changing a magnetic field generated by the magnetic field generation unit is greater when switching according to the switching condition than when switching based on the determination result by the determination unit.

In the guidance device, the guidance controller is configured to continuously change the magnetic field generated by the magnetic field generation unit such that an amount of change in the magnetic field per unit time in the fourth control mode is greater than an amount of change in the magnetic field per unit time in the second control mode when switching to the third control mode based on the determination result by the determination unit.

In the guidance device, the second control mode includes: a fifth control mode that continuously changes the magnetic field generated by the magnetic field generation unit in a direction in which the capsule medical device shifts from a state in which the capsule medical device is in contact with the boundary surface to a state in which the capsule medical device is not in contact with the boundary surface; and a sixth control mode that continuously changes the magnetic field generated by the magnetic field generation unit so that the capsule medical device shifts to the state in which the capsule medical device is not in contact with the boundary surface based on the determination result by the determination unit after the fifth control mode. The guidance controller is configured to control the magnetic field generation unit such that an amount of change in the magnetic field per unit time in the fifth control mode is greater than an amount of change in the magnetic field per unit time in the sixth control mode.

The guidance device further includes a position detection unit configured to detect a position of the capsule medical device in the subject and output position information. The determination unit is configured to determine whether or not the capsule medical device is in contact with the boundary surface, based on the position information.

In the guidance device, the capsule medical device includes an imaging unit for imaging an inside of the subject to generate image data, and a transmitting unit for wirelessly transmitting the image data. The determination unit is configured to determine whether or not the capsule medical device is in contact with the boundary surface based on the image data wirelessly transmitted from the capsule medical device.

The guidance device further includes an operation input unit configured to input instruction information for changing at least one of position and posture of the capsule medical device into the guidance controller according to an operation performed from outside. The guidance controller is configured to determine whether or not to perform control based on the instruction information according to a control mode being performed.

In the guidance device, the guidance controller is configured to determine not to perform the control based on the instruction information even when the instruction information is inputted from the operation input unit while the second control mode is being performed.

The guidance device further includes an operation input unit configured to input instruction information for changing at least one of position and posture of the capsule medical device into the guidance controller according to an operation performed from outside. The guidance controller is configured to determine not to perform control based on the instruction information even when the instruction information is inputted from the operation input unit while the fourth control mode is being performed.

The guidance device further includes an operation input unit configured to input instruction information for changing at least one of position and posture of the capsule medical device into the guidance controller according to an operation performed from outside. The guidance controller is configured to determine not to perform control based on the instruction information even when the instruction information is inputted from the operation input unit while the sixth control mode is being performed.

The guidance device further includes a display unit configured to display a sign showing a state whether or not to perform control based on the instruction information. The display unit is configured to switch the state of the sign according to a control mode being performed by the guidance controller.

A capsule medical device guidance system according to the invention includes the guidance device and the capsule medical device.

### Advantageous Effects of Invention

According to the present invention, because a guidance controller continuously changes a magnetic field generated by a magnetic field generation unit, in a second control mode, from when a capsule medical device is in contact with a boundary surface until the capsule medical device becomes apart from the boundary surface, it is possible to stably guide the capsule medical device in liquid regardless of differences between capsule medical devices.

### Brief Description of Drawings

FIG. 1 is a diagram illustrating a configuration example of a capsule medical device guidance system according to a first embodiment of the present invention.
FIG. 2 is a schematic diagram illustrating an example of an internal structure of a capsule endoscope illustrated in FIG. 1.
FIG. 3 is a schematic diagram illustrating a configuration example of an external appearance of a guidance device illustrated in FIG. 1.
FIG. 4 is a schematic diagram for explaining an installation state of an extracorporeal permanent magnet illustrated in FIG. 1.
FIG. 5 is a schematic diagram for explaining a guidance method in a case in which the capsule endoscope is translated in a plane.
FIG. 6 is a schematic diagram for explaining a guidance method in a case in which the capsule endoscope is translated in a vertical direction.
FIG. 7 is a schematic diagram for explaining a guidance method in a case in which an inclination angle and an azimuth angle of the capsule endoscope are changed.
FIG. 8 is a schematic diagram illustrating an example of a screen displayed on a display unit illustrated in FIG. 1.
FIG. 9 is a flowchart illustrating an operation of the capsule medical device guidance system illustrated in FIG. 1.
FIG. 10 is a schematic diagram for explaining a guidance method of the capsule endoscope of the capsule medical device guidance system illustrated in FIG. 1.
FIG. 11 is a schematic diagram illustrating an example of a screen displayed on the display unit while the capsule medical device guidance system illustrated in FIG. 1 is operating.
FIG. 12 is a schematic diagram illustrating an example of a screen displayed on the display unit while the capsule medical device guidance system illustrated in FIG. 1 is operating.
FIG. 13 is a schematic diagram for explaining a guidance method of a capsule endoscope of a capsule medical device guidance system according to a first modified example of the first embodiment of the present invention.
FIG. 14 is a diagram illustrating a configuration example of a capsule medical device guidance system according to a second modified example of the first embodiment of the present invention.
FIG. 15 is a flowchart illustrating an operation of a capsule medical device guidance system according to a second embodiment of the present invention.
FIG. 16 is a schematic diagram for explaining a guidance method of a capsule endoscope of the capsule medical device guidance system according to the second embodiment of the present invention.
FIG. 17 is a flowchart illustrating an operation of a capsule medical device guidance system according to a third embodiment of the present invention.
FIG. 18 is a schematic diagram for explaining a guidance method of a capsule endoscope of the capsule medical device guidance system according to the third embodiment of the present invention.
FIG. 19 is a schematic diagram for explaining a guidance method of the capsule endoscope of the capsule medical device guidance system according to the third embodiment of the present invention.
FIG. 20 is a schematic diagram for explaining a guidance method of a capsule endoscope of a capsule medical device guidance system according to a fourth embodiment of the present invention.

### Description of Embodiments

Hereinafter, a guidance device and a capsule medical device guidance system according to the embodiments will be described with reference to the drawings. In the description below, a capsule endoscope which is introduced into a subject through mouth and captures images inside a digestive tract of the subject is exemplified as a form of a capsule medical device to be guided by the capsule medical device guidance system according to the embodiments. However, the present invention is not limited by the embodiments. The present invention can be applied to guidance of various capsule type medical devices such as, for example, a capsule endoscope that moves in lumen from esophagus to anus of the subject, a capsule medical device that delivers a medicine or the like into the subject, and a capsule medical device including a PH sensor that measures PH in the subject.

In the description below, the drawings only schematically illustrate the shapes, sizes, and positional relationships so that the contents of the present invention can be understood. Therefore, the present invention is not limited to only the shapes, sizes, and positional relationships illustrated in the drawings. The same reference signs are used to designate the same elements throughout the drawings.

### (First Embodiment)

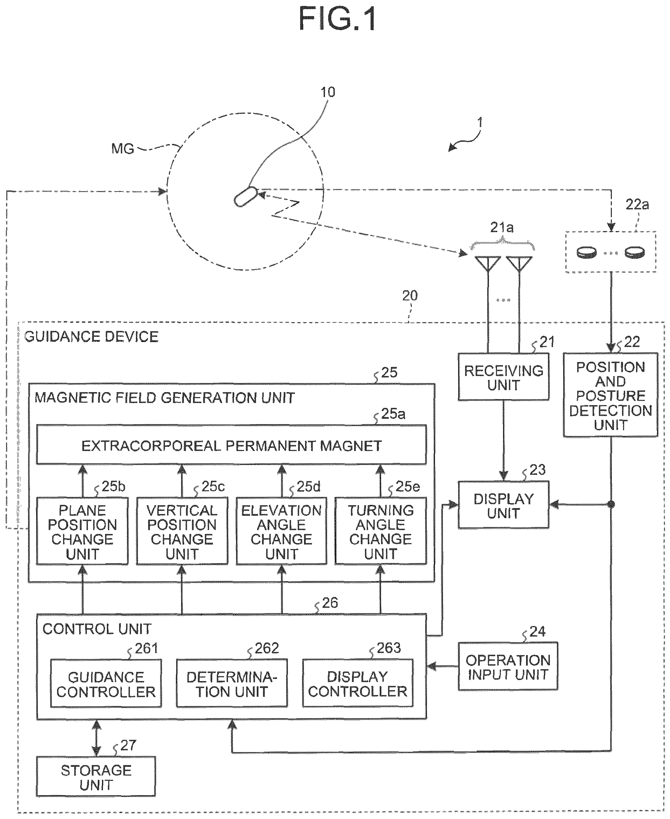

FIG. 1 is a diagram illustrating a configuration example of a capsule medical device guidance system according to a first embodiment of the present invention. As illustrated in FIG. 1, the capsule medical device guidance system 1 according to the first embodiment of the present invention is a capsule medical device to be introduced into a body cavity of a subject and includes a capsule endoscope 10 in which a permanent magnet is provided and a guidance device 20 that generates a magnetic field MG and guides the capsule endoscope 10 introduced into the subject.

The capsule endoscope 10 is introduced inside an organ of the subject along with a predetermined liquid by oral ingestion or the like, then moves inside a digestive tract, and finally is discharged to the outside of the subject. Meanwhile, the capsule endoscope 10 drifts in the liquid introduced inside an organ such as a stomach of the subject, sequentially captures in-vivo images while being guided by the magnetic field MG, and sequentially wirelessly transmits image data corresponding to the in-vivo images acquired by the image capturing.

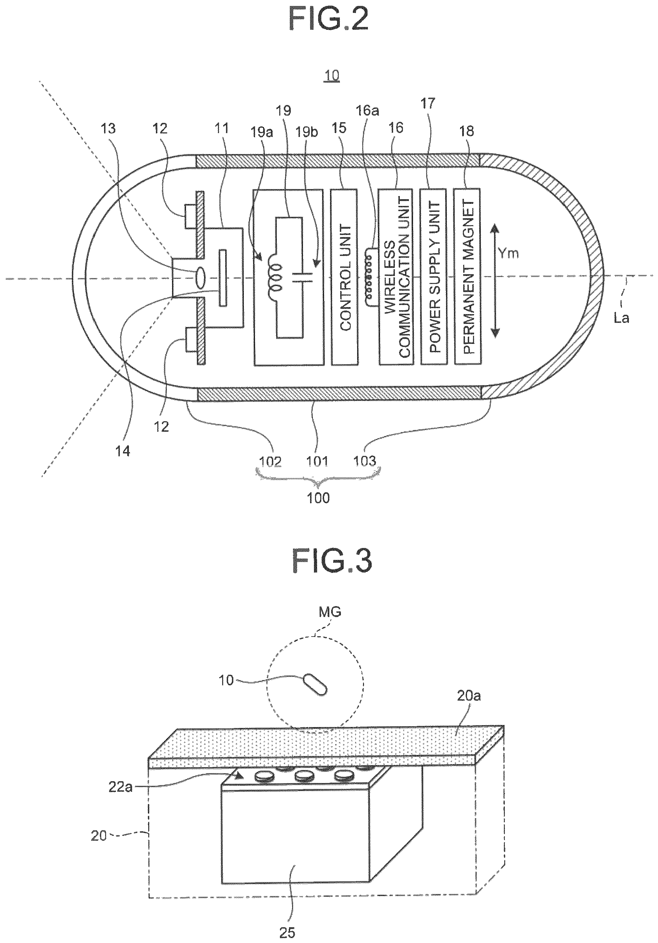

FIG. 2 is a schematic diagram illustrating an example of an internal structure of the capsule endoscope 10. As illustrated in FIG. 2, the capsule endoscope 10 includes a capsule-shaped casing 100 that is an outer packaging formed into a size that can be easily introduced into an organ of the subject, an imaging unit 11 that captures an image signal obtained by capturing an image inside the subject, a control unit 15 that processes the image signal outputted from the imaging unit 11 and controls each component of the capsule endoscope 10, a wireless communication unit 16 that wirelessly transmits the image signal processed by the control unit 15 to the outside of the capsule endoscope 10, a power supply unit 17 that supplies power to each component of the capsule endoscope 10, a permanent magnet 18 that enables guidance performed by the guidance device 20, and a position detection magnetic field generation unit 19 that generates a position detection magnetic field that is a magnetic field used to detect the position of the capsule endoscope 10.

The capsule-shaped casing 100 is an outer casing formed into a size that can be introduced into an organ of the subject. The capsule-shaped casing 100 includes a tubular casing 101 and dome-shaped casings 102 and 103. The capsule-shaped casing 100 is formed by closing open ends at both sides of the tubular casing 101 with the dome-shaped casings 102 and 103. The tubular casing 101 and the dome-shaped casing 103 are colored casings that are substantially opaque to visible light. The dome-shaped casing 102 is an optical member that is transparent to light of a predetermined wavelength band such as visible light. As illustrated in FIG. 2, the capsule-shaped casing 100 internally includes the imaging unit 11, the control unit 15, the wireless communication unit 16, the power supply unit 17, the permanent magnet 18, and the position detection magnetic field generation unit 19 in a liquid-tight manner.

The imaging unit 11 has an illumination unit 12 such as an LED, an optical system 13 such as a condenser lens, and an image sensor 14 such as a CMOS image sensor or a CCD. The illumination unit 12 emits illumination light such as white light to an imaging visual field of the image sensor 14 and illuminates a subject in the imaging visual field through the dome-shaped casing 102. The optical system 13 condenses reflection light from the imaging visual field on an imaging surface of the image sensor 14 and forms an image. The image sensor 14 converts the reflection light from the imaging visual field that is received on the imaging surface into an electrical signal and outputs the electrical signal as an image signal.

The control unit 15 controls operations of the imaging unit 11 and the wireless communication unit 16 and controls input/output of signals between these components. Specifically, the control unit 15 causes the image sensor 14 to capture an image of the subject in the imaging visual field illuminated by the illumination unit 12 and performs predetermined signal processing on the image signal outputted from the image sensor 14. Further, the control unit 15 causes the wireless communication unit 16 to sequentially wirelessly transmits the image signals, on which the signal processing is performed, along the time series.

The wireless communication unit 16 acquires the image signal of in-vivo image outputted by the imaging unit 11 from the control unit 15, performs modulation processing or the like on the image signal, and generates a wireless signal. The wireless communication unit 16 includes an antenna 16a for transmitting the wireless signal and wirelessly transmits the generated wireless signal through the antenna 16a.

The power supply unit 17 is a power storage unit such as a button-shaped battery and a capacitor and has a switch unit such as a magnetic switch and an optical switch. When the power supply unit 17 has a magnetic switch, an ON/OFF state of power supply is switched by a magnetic field applied from the outside. When the power supply is in the ON state, power of the power storage unit is appropriately supplied to each component of the capsule endoscope 10, that is, the imaging unit 11, the control unit 15, and the wireless communication unit 16. When the power supply unit 17 is in the OFF state, the power supply unit 17 stops power supply to each component of the capsule endoscope 10.

The permanent magnet 18 enables guidance of the capsule endoscope 10 performed by the magnetic field MG generated by a magnetic field generation unit 25 described later. The permanent magnet 18 is arranged and fixed inside the capsule-shaped casing 100 so that a magnetization direction Ym has an inclination with respect to a long axis La. In the first embodiment, the permanent magnet 18 is arranged so that the magnetization direction Ym is perpendicular to the long axis La. The permanent magnet 18 moves following the magnetic field applied from the outside. As a result, the capsule endoscope 10 is guided by the magnetic field generation unit 25.

The position detection magnetic field generation unit 19 forms a part of a resonance circuit and includes a marker coil 19a that generates a magnetic field when an electric current flows therein and a capacitor 19b that forms a resonance circuit along with the marker coil 19a. The position detection magnetic field generation unit 19 receives a power supply from the power supply unit 17 and generates a position detection magnetic field.

As illustrated in FIG. 1, the guidance device 20 includes a receiving unit 21 that performs wireless communication with the capsule endoscope 10 and receives a wireless signal including an image signal transmitted from the capsule endoscope 10, a position and posture detection unit 22 that detects a position and a posture of the capsule endoscope 10 in the subject based on the position detection magnetic field generated by the position detection magnetic field generation unit 19 of the capsule endoscope 10, a display unit 23 that acquires the image signal from the wireless signal received by the receiving unit 21, performs predetermined signal processing on the image signal to display the in-vivo image, and displays information of the position and the posture of the capsule endoscope 10 in the subject, an operation input unit 24 that receives an input such as information instructing various operations in the capsule medical device guidance system 1, the magnetic field generation unit 25 that generates a magnetic field for guiding the capsule endoscope 10, a control unit 26 that controls these units, and a storage unit 27 that stores image data of the in-vivo image and the like.

FIG. 3 is a schematic diagram illustrating a configuration example of an external appearance of the guidance device 20. As illustrated in FIG. 3, the guidance device 20 is provided with a bed 20a as a mounting table on which the subject is mounted. At least the magnetic field generation unit 25 that generates the magnetic field MG and a plurality of sensing coils 22a that detect the position detection magnetic field generated by the position detection magnetic field generation unit 19 are arranged below the bed 20a.

The receiving unit 21 includes a plurality of receiving antennas 21a and sequentially receives wireless signals from the capsule endoscope 10 through the receiving antennas 21a. The receiving unit 21 selects an antenna where a received electric field strength is the highest from among the receiving antennas 21a and performs demodulation processing and the like on the wireless signal received from the capsule endoscope 10 through the selected antenna, thereby extracting an image signal from the wireless signal and outputting the image signal to the display unit 23.

The plurality of sensing coils 22a are arranged on a flat panel arranged in parallel with the supper surface of the bed 20a. Each sensing coil 22a is, for example, a tubular coil having a coil spring shape. The sensing coil 22a receives the magnetic field generated by the position detection magnetic field generation unit 19 of the capsule endoscope 10 and outputs a detection signal.

The position and posture detection unit 22 acquires a plurality of detection signals respectively outputted from the plurality of sensing coils 22a and performs signal processing such as waveform shaping, amplification, A/D conversion, and FFT, thereby extracting magnetic field information such as amplitude and phase of the position detection magnetic field. Further, the position and posture detection unit 22 calculates the position and the posture of the capsule endoscope 10 based on the magnetic field information and outputs the position and the posture as position information.

The detection method of the position and the posture of the capsule endoscope 10 is not limited to the method that uses the position detection magnetic field described above. For example, it is possible to detect the position and the posture of the capsule endoscope 10 based on strength distribution of the wireless signals received by the receiving unit 21. As an example, it is possible to obtain the position of the capsule endoscope 10 by appropriately setting an initial value of the position of the capsule endoscope 10 and repeating processing for calculating an estimate value of the position by Gauss-Newton method until a difference between the calculated estimate value and the previous estimate value becomes smaller than or equal to a predetermined value (for example, see JP 2007-283001 A).

The display unit 23 has a screen made from any sort of display such as a liquid crystal display and displays an in-vivo image based on the image signal inputted from the receiving unit 21, the position information of the capsule endoscope 10, and other various kinds of information on the screen.

The operation input unit 24 inputs operation input information that is instruction information for controlling the position or the posture of the capsule endoscope 10 and instruction information for changing a control mode that controls a magnetic field for guiding the capsule endoscope 10 into the control unit 26 according to an operation performed by a user from the outside. The operation input unit 24 is formed of input devices such as a joystick, an operation console including various buttons and various switches, and a keyboard.

Specifically, the operation input information includes information related to a translation operation that translates the capsule endoscope 10 in a horizontal direction or a vertical direction (a gravity direction), an inclination angle change operation that changes an inclination angle of the long axis La of the capsule endoscope 10 with respect to the vertical direction, and an azimuth angle change operation that changes an azimuth angle of visual field of the imaging unit 11 provided in the capsule endoscope 10, that is, an angle around an axis in the vertical direction.

The magnetic field generation unit 25 generates the magnetic field MG for relatively changing the position, the inclination angle, and the azimuth angle of the capsule endoscope 10 introduced into the subject with respect to the subject. The magnetic field generation unit 25 has an extracorporeal permanent magnet 25a that generates the magnetic field MG, and a plane position change unit 25b, a vertical position change unit 25c, an elevation angle change unit 25d, and a turning angle change unit 25e which change the position and the posture of the extracorporeal permanent magnet 25a.

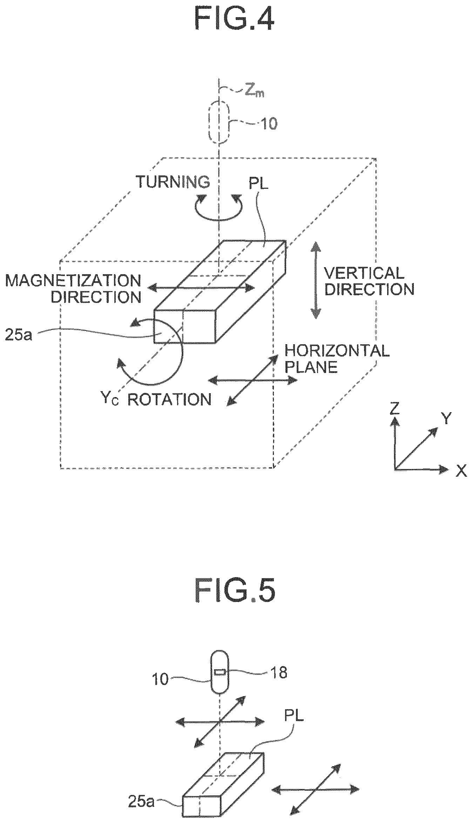

FIG. 4 is a schematic diagram for explaining an installation state of the extracorporeal permanent magnet 25a. As illustrated in FIG. 4, the extracorporeal permanent magnet 25a is formed of, for example, a bar magnet having a rectangular parallelepiped shape. In an initial state, the extracorporeal permanent magnet 25a is arranged so that one surface of four surfaces in parallel with the magnetization direction of the extracorporeal permanent magnet 25a is in parallel with a horizontal plane that is a surface perpendicular to the vertical direction. Hereinafter, the arrangement of the extracorporeal permanent magnet 25a when the extracorporeal permanent magnet 25a is in the initial state is referred to as a reference arrangement. Further, a surface which is one of the four surfaces in parallel with the magnetization direction of the extracorporeal permanent magnet 25a and is a surface facing the capsule endoscope 10 is referred to as a capsule facing surface PL.

The plane position change unit 25b translates the extracorporeal permanent magnet 25a in the horizontal plane. Specifically, the plane position change unit 25b moves the extracorporeal permanent magnet 25a in the horizontal plane in a state in which relative positions of two magnetic poles magnetized in the extracorporeal permanent magnet 25a are secured.

The vertical position change unit 25c is a translation mechanism that translates the extracorporeal permanent magnet 25a along the vertical direction (Z direction). Specifically, the vertical position change unit 25c moves the extracorporeal permanent magnet 25a along the vertical direction in a state in which relative positions of two magnetic poles magnetized in the extracorporeal permanent magnet 25a are secured.

The elevation angle change unit 25d is a rotation mechanism that changes an angle of the magnetization direction of the extracorporeal permanent magnet 25a with respect to the horizontal plane by rotating the extracorporeal permanent magnet 25a in a vertical plane including the magnetization direction of the extracorporeal permanent magnet 25a. In other words, the elevation angle change unit 25d rotates the extracorporeal permanent magnet 25a with respect to an axis Y_{c} in a Y direction which is in parallel with the capsule facing surface PL, is perpendicular to the magnetization direction, and passes through the center of the extracorporeal permanent magnet 25a. Hereinafter, an angle between the extracorporeal permanent magnet 25a and the horizontal plane is defined as an elevation angle.

The turning angle change unit 25e rotates the extracorporeal permanent magnet 25a with respect to a vertical axis Zₘ that passes through the center of the extracorporeal permanent magnet 25a. Hereinafter, the rotation movement of the extracorporeal permanent magnet 25a with respect to the vertical axis Zₘ is referred to as a turning movement. Further, an angle by which the extracorporeal permanent magnet 25a turns with respect to the reference arrangement is defined as a turning angle.

The control unit 26 includes a guidance controller 261 that controls the operation of the magnetic field generation unit 25, a determination unit 262 that determines whether or not the capsule endoscope 10 is in contact with the boundary surface of the liquid introduced into the subject, and a display controller 263 that controls a display state of images and various information displayed on the display unit 23.

The guidance controller 261 causes the magnetic field generation unit 25 to generate a magnetic field for guiding the capsule endoscope 10 by a set control mode based on the position information of the capsule endoscope 10 inputted from the position and posture detection unit 22 and the instruction information inputted from the operation input unit 24. The control mode for the magnetic field generation unit 25 includes a first control mode that generates a magnetic field that guides the capsule endoscope 10 in a direction in which the capsule endoscope 10 moves and comes into contact with an upper or a lower boundary surface of the liquid introduced into the subject, a second control mode that continuously changes the magnetic field so that the capsule endoscope 10 shifts from a state in which the capsule endoscope 10 is in contact with the boundary surface to a state in which the capsule endoscope 10 is not in contact with the boundary surface, and a third control mode that controls the magnetic field so that the capsule endoscope 10 is held in the liquid. Hereinafter, although the surface of the liquid introduced into the subject is referred to as a water surface, "in the liquid" is referred to as "in the water", and the bottom of the liquid is referred to as a water bottom, it is assumed that the liquid may be other than water (for example, the liquid may be a physiological salt solution or the like). In the first embodiment, the guidance controller 261 performs a water bottom mode that causes the capsule endoscope 10 to come into contact with the water bottom as the first control mode, performs an in-the-water mode that causes the capsule endoscope 10 to detach from the water bottom as the second control mode, and performs a floating mode that guides the capsule endoscope 10 according to the operation input information while maintaining a state in which the capsule endoscope 10 is floating in the water as the third control mode.

The determination unit 262 determines whether or not the capsule endoscope 10 is in contact with the water bottom when the control mode is switched from the water bottom mode to the in-the-water mode. Whether or not the capsule endoscope 10 is in contact with the water bottom may be determined by image processing on an in-vivo image generated by imaging performed by the imaging unit 11 of the capsule endoscope 10 or may be determined based on the position information outputted from the position and posture detection unit 22 that detects the position of the capsule endoscope 10.

The display controller 263 generates a screen of a predetermined format including an in-vivo image based on an image signal received by the receiving unit 21, the position information of the capsule endoscope 10, and other various information, and causes the display unit 23 to display the screen.

The storage unit 27 includes a storage medium such as flash memory or a hard disk which rewritably stores information and a write/read device that writes and reads information to and from the storage medium. The storage unit 27 stores image data of in-vivo images of the subject captured by the capsule endoscope 10 and information such as various programs and parameters for the control unit 26 to control each unit of the guidance device 20.

Next, a method of guiding the position and the posture of the capsule endoscope 10 by using the magnetic field generated by the magnetic field generation unit 25 will be described. FIG. 5 is a schematic diagram for explaining a guidance method in a case in which the capsule endoscope 10 is translated in a horizontal plane. When translating the capsule endoscope 10 in the horizontal plane, a magnetic field is generated which generates a magnetic attracting force in a direction in which the capsule endoscope 10 is restrained to a specific position in the horizontal plane and the magnetic field is applied to the permanent magnet 18 of the capsule endoscope 10. The specific position is referred to as a restraining position. As illustrated in FIG. 5, the capsule endoscope 10 is restrained by attracting the permanent magnet 18 to the restraining position, and in this state, the extracorporeal permanent magnet 25a is moved in the horizontal plane by the plane position change unit 25b. Thereby, the capsule endoscope 10 is translated in the horizontal plane.

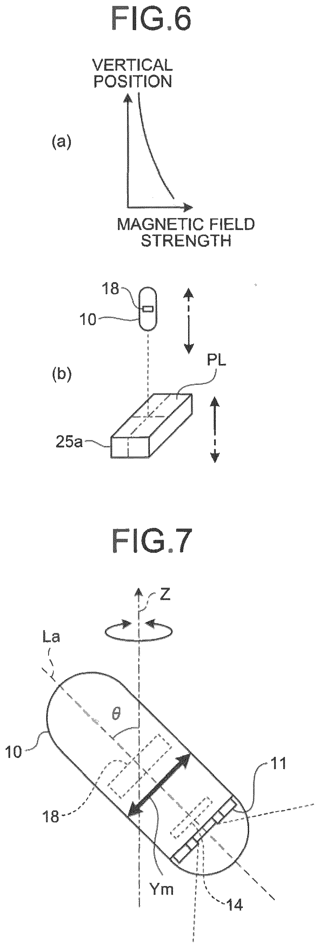

FIG. 6 is a schematic diagram for explaining a guidance method in a case in which the capsule endoscope 10 is translated in the vertical direction. When translating the capsule endoscope 10 in the vertical direction, as illustrated in FIG. 6(a), a magnetic field whose distribution of magnetic field gradients changes according to a distance in a direction perpendicular to the capsule facing surface PL is generated and applied to the permanent magnet 18 of the capsule endoscope 10. Specifically, the extracorporeal permanent magnet 25a is moved in the vertical direction by the vertical position change unit 25c and a distance between the extracorporeal permanent magnet 25a and the permanent magnet 18 is changed. Thereby, as illustrated in FIG. 6(b), the capsule endoscope 10 is translated in the vertical direction. Here, in the distribution of the magnetic field generated by the rectangular parallelepiped extracorporeal permanent magnet 25a as illustrated in FIGS. 4 to 6, the restraining position of the capsule endoscope 10 is on a line which is perpendicular to the capsule facing surface PL and passes through the center of the extracorporeal permanent magnet 25a.

FIG. 7 is a schematic diagram for explaining a guidance method in a case in which the inclination angle and the azimuth angle of the capsule endoscope 10 are changed. When changing an inclination angle θ of the long axis La of the capsule endoscope 10 with respect to the vertical direction, the capsule endoscope 10 is restrained at the restraining position and the elevation angle is changed by rotating the extracorporeal permanent magnet 25a around the axis Y_{c} by the elevation angle change unit 25d. Thereby, the inclination angle θ of the capsule endoscope 10 is changed. When changing the azimuth angle of the capsule endoscope 10, the capsule endoscope 10 is restrained at the restraining position and the extracorporeal permanent magnet 25a is rotated around the vertical axis Zₘ that passes through the center of the extracorporeal permanent magnet 25a by the turning angle change unit 25e. Thereby, the capsule endoscope 10 is rotated around the Z axis and the azimuth angle of the capsule endoscope 10 is changed.

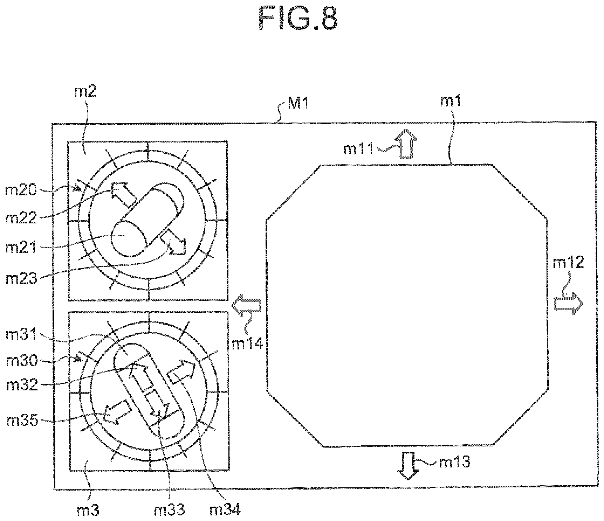

FIG. 8 is a schematic diagram illustrating an example of a screen displayed on the display unit 23. A screen M1 illustrated in FIG. 8 includes an image display area m1 in which an in-vivo image acquired by the capsule endoscope 10 is displayed and posture diagrams m2 and m3 that illustrate the posture of the capsule endoscope 10 in the subject. In the first embodiment, as illustrated by the image display area m1, the four corners of the rectangular in-vivo image are masked out.

The image display area m1 is an area in which an in-vivo image is displayed based on the image signal sequentially inputted from the receiving unit 21. Operation input arrows m11 to m14, which are marks indicating a direction of an operation input that translates the capsule endoscope 10, are displayed around the image display area m1.

The posture diagram m2 illustrates a posture of the capsule endoscope 10 in the horizontal plane and includes a scale m20 indicating the azimuth angle, a model diagram m21 illustrating the posture of the capsule endoscope 10, and operation input arrows m22 and m23 which are marks indicating a direction of an operation input that turns the capsule endoscope 10.

The posture diagram m3 illustrates a posture of the capsule endoscope 10 in a vertical plane and includes a scale m30 indicating the inclination angle, a model diagram m31 illustrating the posture of the capsule endoscope 10, operation input arrows m32 and m33 which are marks indicating a direction of an operation input that translates the capsule endoscope 10 in a long axis direction, and operation input arrows m34 and m35 which are marks indicating a direction of an operation input that inclines the capsule endoscope 10.

The operation input arrows m11 to m14, m22, m23, and m32 to m35 are set so as to be displayed in different states according to the control mode being performed by the control unit 26 and the propriety of the guidance of the capsule endoscope 10. In the first embodiment, colors of the operation input arrows m11 to m14, m22, m23, and m32 to m35 are changed. As an example, while waiting for an operation input to the capsule endoscope 10, the operation input arrows m11 to m14, m22, m23, and m32 to m35 are displayed in white and operation input arrows of a direction in which an operation input to the capsule endoscope 10 is performed are displayed in yellow. While not accepting an operation input to the capsule endoscope 10, it is set so that the operation input arrows m11 to m14, m22, m23, and m32 to m35 are not displayed.

The operation input information inputted from the operation input unit 24 is reflected on a control signal outputted from the control unit 26 when the control unit 26 controls the magnetic field generation unit 25. Therefore, it can be considered that the postures of the capsule endoscope 10 in the model diagrams m21 and m31 displayed in the posture diagrams m2 and m3 are substantially the same as the posture of the actual capsule endoscope 10 in the subject.

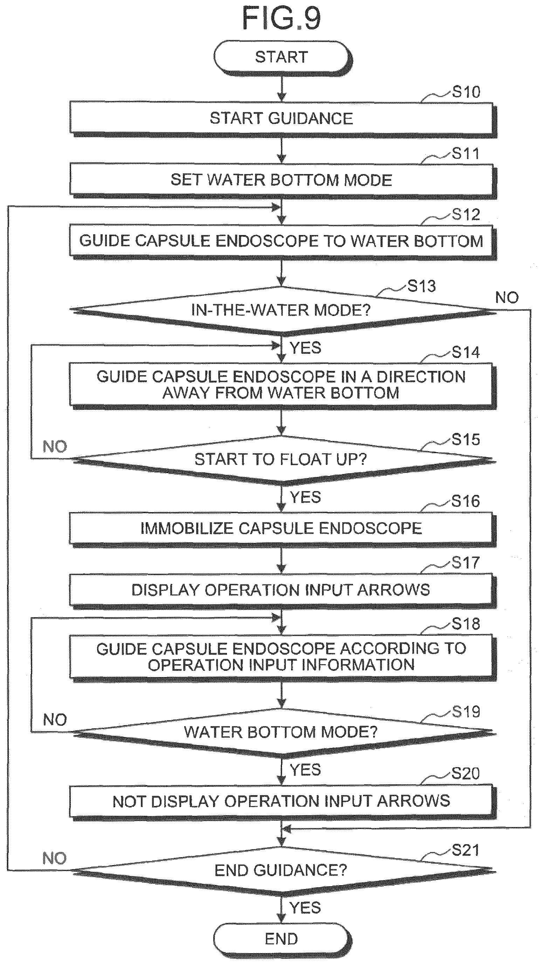

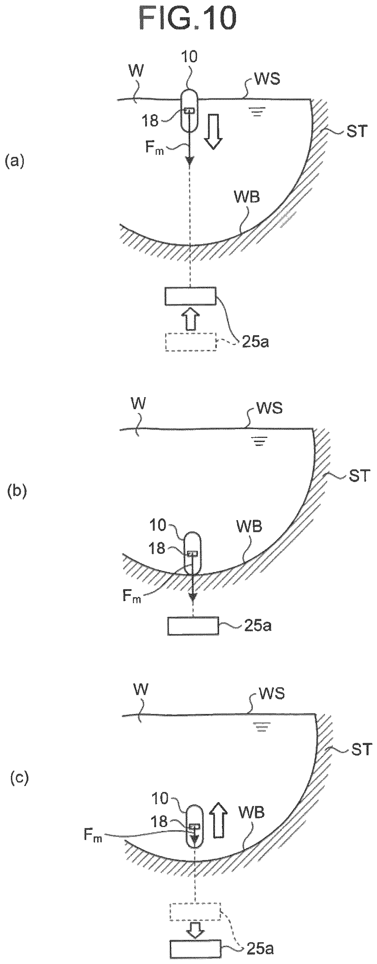

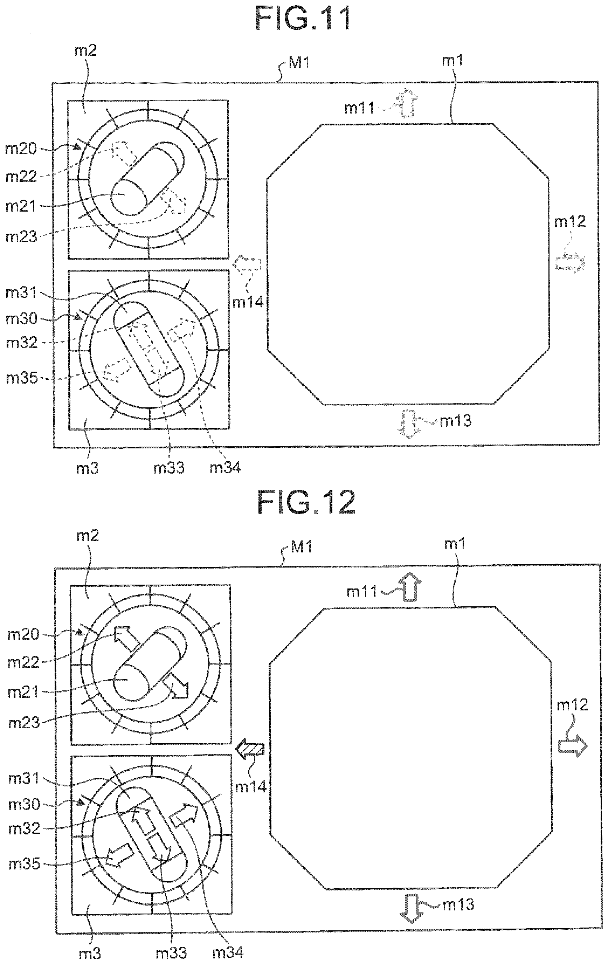

Next, an operation of the capsule medical device guidance system (hereinafter also referred to as simply a system) 1 will be described. FIG. 9 is a flowchart illustrating the operation of the system 1. FIG. 10 is a schematic diagram for explaining a guidance method of the capsule endoscope 10 in the system 1. FIGS. 11 and 12 are schematic diagrams illustrating an example of a screen displayed on the display unit 23 while the system 1 is operating.

As illustrated in FIG. 10, in the system 1, an observation is performed in a state in which the capsule endoscope 10 is floated in a liquid W introduced into an organ ST of the subject by oral ingestion or the like. The liquid W is a liquid harmless to a human body, such as, for example, water and a physiological salt solution.

In the first embodiment, it is assumed that the capsule endoscope 10 has a specific weight smaller than that of the liquid W and is designed to float on the liquid W in a state in which the guidance is not performed by the magnetic field generation unit 25. In this case, it is possible to stop the capsule endoscope 10 at a desired position in the liquid W by a balance among a buoyant force of the capsule endoscope 10 with respect to the liquid W, a gravitational force applied to the capsule endoscope 10, and a magnetic attracting force applied to the permanent magnet 18 by the magnetic field generated by the magnetic field generation unit 25, and observe the inside of the organ ST.

In step S10, when a signal instructing a start of guidance of the capsule endoscope 10 is inputted into the control unit 26 from the operation input unit 24, each unit of the guidance device 20 starts operation. Specifically, the receiving unit 21 receives a wireless signal transmitted by the capsule endoscope 10 and starts an operation to extract an image signal from the wireless signal and output the image signal to the display unit 23. The position and posture detection unit 22 starts an operation to detect the position of the capsule endoscope 10 and output position information. The display unit 23 starts display of an in-vivo image based on the image signal outputted by the receiving unit 21.

The control unit 26 starts acquisition of the position information of the capsule endoscope 10 outputted from the position and posture detection unit 22 and starts control to cause the display unit 23 to display the position of the capsule endoscope 10. At this time, as illustrated in FIG. 11, the display controller 263 does not display the operation input arrows m11 to m14, m22, m23, and m32 to m35 in the screen M1 displayed on the display unit 23.

In the next step S11, the control unit 26 sets the control mode to the water bottom mode.

In the next step S12, the guidance controller 261 causes the magnetic field generation unit 25 to generate a magnetic field that guides the capsule endoscope 10 to a water bottom WB. Specifically, as illustrated in (a) of FIG. 10, the extracorporeal permanent magnet 25a is moved upward (in the plus Z direction) which is a direction toward a water surface WS and is moved close to the capsule endoscope 10, so that a magnetic attracting force Fₘ applied to the permanent magnet 18 is increased. Thereby, the sum of the magnetic attracting force Fₘ and the gravitational force applied to the capsule endoscope 10 is greater than the buoyant force of the capsule endoscope 10, so that the capsule endoscope 10 moves toward the water bottom WB. (b) of FIG. 10 illustrates a state in which the capsule endoscope 10 is in contact with the water bottom WB as a result of the above guidance.

In step S13, the control unit 26 determines whether or not instruction information for switching the control mode from the water bottom mode to the in-the-water mode is inputted from the operation input unit 24. When the instruction information for switching the control mode to the in-the-water mode is not inputted (step S13: No), the operation of the system 1 proceeds to step S21 described later.

On the other hand, when the instruction information for switching the control mode to the in-the-water mode is inputted (step S13: Yes), the guidance controller 261 causes the magnetic field generation unit 25 to generate a magnetic field that guides the capsule endoscope 10 in a direction away from the water bottom WB, that is, in a direction in which the capsule endoscope 10 floats up (step S14). Specifically, as illustrated in (c) of FIG. 10, the extracorporeal permanent magnet 25a is moved vertically downward and is distanced from the capsule endoscope 10, so that the magnetic attracting force Fₘ applied to the permanent magnet 18 is reduced. Thereby, the sum of the magnetic attracting force Fₘ and the gravitational force applied to the capsule endoscope 10 is reduced. Accordingly, a drag which the capsule endoscope 10 receives from the water bottom WB is reduced. At this time, the guidance controller 261 moves the extracorporeal permanent magnet 25a slowly and suppresses a change rate of the magnetic field (the magnetic attracting force Fₘ) so that the capsule endoscope 10 can be immediately immobilized when the capsule endoscope 10 starts to float up from the water bottom WB.

In step S15, the determination unit 262 determines whether or not the capsule endoscope 10 detaches from the water bottom WB and starts to float up. For example, when a change of an in-vivo image is detected by image processing such as pattern matching on the in-vivo image generated based on an image signal transmitted from the capsule endoscope 10 and it is determined that the capsule endoscope 10 is not in contact with the water bottom WB, it can be determined that the capsule endoscope 10 starts to float up. Alternatively, when a difference between a Z coordinate value when the capsule endoscope 10 remains still at the water bottom WB and a Z coordinate value of the current capsule endoscope 10 becomes greater than or equal to a predetermined value, it may be determined that the capsule endoscope 10 starts to float up.

When the capsule endoscope 10 does not float up from the water bottom WB (step S15: No), the guidance controller 261 continues the guidance of the capsule endoscope 10 in a direction in which the capsule endoscope 10 moves away from the water bottom WB (step S14).

On the other hand, when the capsule endoscope 10 detaches from the water bottom WB and starts to float up (step S15: Yes), the control mode of the guidance controller 261 switches to the floating mode and the guidance controller 261 causes the magnetic field generation unit 25 to generate a magnetic field that immobilizes the capsule endoscope 10 in the water (step S16). Here, at the moment when the capsule endoscope 10 detaches from the water bottom WB, the magnitude of the drag of the water bottom WB to the capsule endoscope 10 becomes zero and it can be said that the moment is a moment when the sum of the magnetic attracting force Fₘ applied to the capsule endoscope 10 and the buoyant force is balanced with the gravitational force. Therefore, it is possible to immobilize the capsule endoscope 10 in the water by stopping the movement of the extracorporeal permanent magnet 25a at this time.

In the next step S17, the display controller 263 displays the operation input arrows m11 to m14, m22, m23, and m32 to m35 in the screen M1 of the display unit 23. In the first embodiment, specifically, the display controller 263 displays the operation input arrows m11 to m14, m22, m23, and m32 to m35 in white. Thereby, a user can recognize that an operation input for guiding the capsule endoscope 10 is possible.

In step S18, the guidance controller 261 performs control to guide the capsule endoscope 10 according to the operation input information inputted from the operation input unit 24 while feeding back the position information of the capsule endoscope 10. Specifically, the guidance controller 261 performs control to translate the capsule endoscope 10 in the horizontal direction or the vertical direction in the water or change the inclination angle and the azimuth angle of the capsule endoscope 10. In this case, as illustrated in FIG. 12, the display controller 263 displays the operation input arrow in a direction in which an operation input is performed in a color different from that of the operation input arrows in the other directions. In the first embodiment, specifically, the operation input arrow m14 in a direction in which an operation input is performed is displayed in yellow. In FIG. 12, a difference between colors is indicated by a difference between patterns.

In step S19, the control unit 26 determines whether or not instruction information for switching the control mode from the in-the-water mode to the water bottom mode is inputted from the operation input unit 24. When the instruction information for switching the control mode to the water bottom mode is not inputted (step S19: No), the guidance controller 261 continues the guidance of the capsule endoscope 10 based on the operation input information (step S18).

On the other hand, when the instruction information for switching the control mode to the water bottom mode is inputted (step S19: Yes), the display controller 263 does not display the operation input arrows m11 to m14, m22, m23, and m32 to m35 in the screen M1 of the display unit 23 (step S20). Thereby, a user can recognize that reception of operation input for guiding the capsule endoscope 10 is suspended.

In the next step S21, the control unit 26 determines whether or not instruction information for ending the guidance of the capsule endoscope 10 is inputted from the operation input unit 24. When the instruction information for ending the guidance is not inputted (step S21: No), the operation of the system 1 returns to step S12. On the other hand, when the instruction information for ending the guidance is inputted (step S21: Yes), the operation of the system 1 ends.

As described above, according to the first embodiment, when switching the control mode from the water bottom mode to the in-the-water mode, the magnetic attracting force Fₘ applied to the permanent magnet 18 is gradually reduced by slowly distancing the extracorporeal permanent magnet 25a from the capsule endoscope 10 while determining whether or not the capsule endoscope 10 detaches from the water bottom WB and starts to float up. Therefore, it is possible to immediately stop the change of the magnetic attracting force Fₘ and immobilize the capsule endoscope 10 when the capsule endoscope 10 detaches from the water bottom WB. Therefore, it is possible to stably guide the capsule endoscope 10 regardless of individual difference of the capsule endoscope 10.

Further, according to the first embodiment, when the control mode is switched to the in-the-water mode, while the capsule endoscope 10 is floating up from the water bottom WB to the in-the-water, the operation input arrows m11 to m14, m22, m23, and m32 to m35 are not displayed in the screen M1. On the other hand, when the capsule endoscope 10 remains still and an operation input for guiding the capsule endoscope 10 can be performed, the operation input arrows m11 to m14, m22, m23, and m32 to m35 are displayed. Therefore, a user can easily know the state of the capsule endoscope in the subject and efficiently perform an operation input of the capsule endoscope 10. An indicator for displaying whether or not an operation input can be performed may be provided instead of switching between displaying and not displaying the operation input arrows m11 to m14, m22, m23, and m32 to m35.

### (First Modified Example)

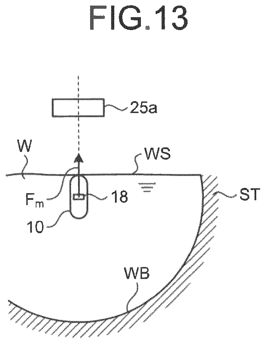

Next, a first modified example of the first embodiment of the present invention will be described. FIG. 13 is a schematic diagram for explaining a guidance method of a capsule endoscope 10 of a capsule medical device guidance system according to the first modified example of the first embodiment of the present invention.

In the first embodiment, the guidance method of the capsule endoscope 10 when the control mode is switched from the water bottom mode to the in-the-water mode is described. However, when the control mode is switched from a water surface mode to the in-the-water mode, it is also possible to guide the capsule endoscope 10 by changing the magnetic field in the same manner.

Specifically, the capsule endoscope 10 is designed so that the capsule endoscope 10 has a specific weight greater than that of the liquid W and the capsule endoscope 10 sinks in the liquid W in a state in which no guidance is performed by the magnetic field generation unit 25. In this case, as illustrated in FIG. 13, the extracorporeal permanent magnet 25a is arranged above the subject and a vertically upward magnetic attracting force Fₘ is applied to the permanent magnet 18 built in the capsule endoscope 10.

After starting the guidance of the capsule endoscope 10, first, the control unit 26 sets the control mode to the water surface mode and causes the magnetic field generation unit 25 to generate a magnetic field that guides the capsule endoscope 10 to the water surface WS. Specifically, the control unit 26 causes the extracorporeal permanent magnet 25a to move downward to be close to the capsule endoscope 10 so that the sum of the magnetic attracting force Fₘ applied to the permanent magnet 18 and the buoyant force of the capsule endoscope 10 is greater than the gravitational force applied to the capsule endoscope 10.

When instruction information for switching the control mode from the water surface mode to the in-the-water mode is inputted from the operation input unit 24, the guidance controller 261 causes the magnetic field generation unit 25 to generate a magnetic field that guides the capsule endoscope 10 in a direction away from the water surface WS, that is, in a direction in which the capsule endoscope 10 sinks. Specifically, the extracorporeal permanent magnet 25a is moved vertically upward and is distanced from the capsule endoscope 10, so that the magnetic attracting force Fₘ applied to the permanent magnet 18 is reduced. Thereby, the sum of the magnetic attracting force Fₘ and the buoyant force of the capsule endoscope 10 is reduced. Accordingly, a drag which the capsule endoscope 10 receives from the water surface WS is reduced. At this time, the guidance controller 261 moves the extracorporeal permanent magnet 25a slowly and suppresses a change rate of the magnetic field (the magnetic attracting force Fₘ) based on a determination result of the determination unit 262 so that the capsule endoscope 10 can be immediately immobilized when the capsule endoscope 10 starts to sink from the water surface WS.

### (Second Modified Example)

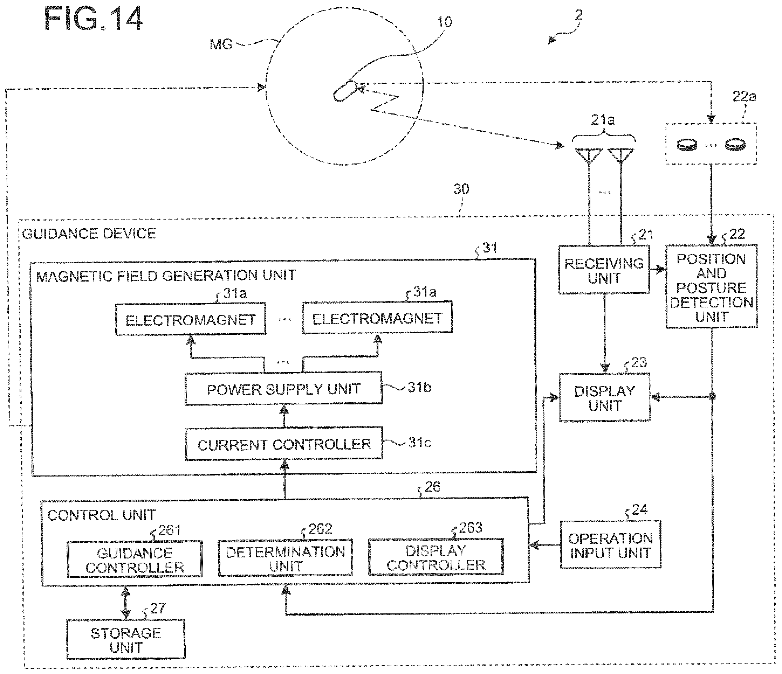

Next, a second modified example of the first embodiment of the present invention will be described. FIG. 14 is a diagram illustrating a configuration example of a capsule medical device guidance system according to the second modified example of the first embodiment of the present invention. As illustrated in FIG. 14, a capsule medical device guidance system 2 according to the second modified example includes a guidance device 30, which has a magnetic field generation unit 31, instead of the guidance device 20 illustrated in FIG. 1.

The magnetic field generation unit 31 includes a plurality of electromagnets 31a, a power supply unit 31b that supplies power to each electromagnet 31a, and a current controller 31c that controls a current to be flown in each electromagnet 31a under control of the control unit 26. The current controller 31c generates a synthetic magnetic field having a restraining position to be applied to the permanent magnet 18 in the capsule endoscope 10 by controlling the magnitude of the current to be supplied to each electromagnet 31a. The components other than the magnetic field generation unit 31 in the capsule medical device guidance system 2 are the same as those in the first embodiment.

The capsule medical device guidance system 2 in the second modified example changes the strength of the synthetic magnetic field formed by these electromagnets 31a by changing the power supplied to each electromagnet 31a when changing the magnetic field for guiding the capsule endoscope 10 in steps S12, S14, and S18 illustrated in FIG. 9.

According to the second modified example, it is possible to change the magnetic field applied to the permanent magnet 18 without providing a mechanical moving mechanism or the like, so that it is possible to realize quick response.

### (Second Embodiment)

Next, a second embodiment of the present invention will be described. A configuration of a capsule medical device guidance system according to the second embodiment of the present invention is the same as that of the first embodiment illustrated in FIGS. 1 to 3, and a control operation performed by the control unit 26 is different from that of the first embodiment.

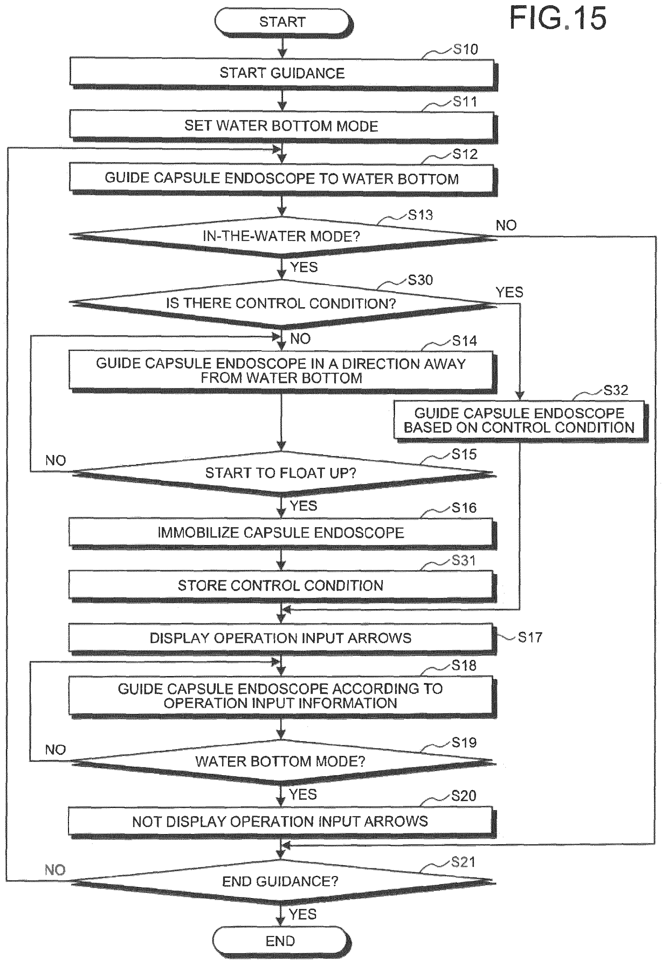

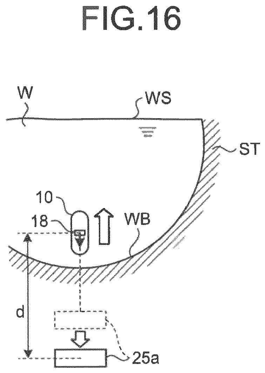

FIG. 15 is a flowchart illustrating an operation of the capsule medical device guidance system (hereinafter also referred to as simply a system) 1 according to the second embodiment of the present invention. FIG. 16 is a schematic diagram for explaining a guidance method of the capsule endoscope 10 in the system 1. Steps S10 to S13 illustrated in FIG. 15 are the same as those in the first embodiment.

In step S30 following step S13, the control unit 26 determines whether or not a control condition for guiding the capsule endoscope 10 in use is stored in the storage unit 27. The control condition is a condition of a position of the extracorporeal permanent magnet 25a for transferring the state of the capsule endoscope 10 from a state in which the capsule endoscope 10 is in contact with the water bottom WB to a state in which the capsule endoscope 10 remains still in the water after detaching from the water bottom WB and floating up. The control condition is represented by a relative position with respect to the capsule endoscope 10. Here, there are individual differences in the size of the permanent magnet 18 built in the capsule endoscope 10 and the volume and the mass of the capsule endoscope 10 for each capsule endoscope 10. Therefore, the control condition is not stored in the storage unit 27 immediately after starting an examination using the capsule endoscope 10.

When the control condition is not stored in the storage unit 27 (step S30: No), the control unit 26 obtains the control condition. In detail, after performing steps S14 to S16 in the same manner as in the first embodiment, in the subsequent step S31, the control unit 26 stores a relative position of the extracorporeal permanent magnet 25a with respect to the capsule endoscope 10 at a time when the capsule endoscope 10 detaches from the water bottom WB and remains still in step S16, specifically, stores a distance d between the capsule endoscope 10 and the extracorporeal permanent magnet 25a illustrated in FIG. 16, into the storage unit 27 as the control condition.

On the other hand, when the control condition is stored in the storage unit 27 in step S30 (step S30: Yes), the guidance controller 261 reads the control condition from the storage unit 27 and guides the capsule endoscope 10 based on the control condition (step S32). In detail, the guidance controller 261 determines a position of the extracorporeal permanent magnet 25a at which the distance between the capsule endoscope 10 and the extracorporeal permanent magnet 25a is close to the distance d as a switching condition of the control mode based on the distance d stored as the control condition. Then, the guidance controller 261 moves the extracorporeal permanent magnet 25a to the position of the determined switching condition, that is, the position close to the distance d.

In this way, the extracorporeal permanent magnet 25a is moved according to the switching condition based on the distance d stored as the control condition. Thereby, it is possible to cause the capsule endoscope 10 to float up from the water bottom WB and remain still in the water. The subsequent steps S17 to S21 are the same as those in the first embodiment.

As described above, according to the second embodiment, when the control mode is switched from the water bottom mode to the in-the-water mode, the control of the magnetic field for causing the capsule endoscope 10 to start to float up by moving the extracorporeal permanent magnet 25a while determining whether or not the capsule endoscope 10 detaches from the water bottom WB and floats up (steps S14 and S15) only has to be performed once after starting the use of the capsule endoscope 10. Thereafter, it is possible to control the magnetic field by using the control condition acquired at the first time. In other words, thereafter, it is possible to more quickly and stably perform the control of the magnetic field for causing the capsule endoscope 10 to float up from the water bottom WB and remain still in the water than in the control of the magnetic field at the first time. Accordingly, it is possible to execute the examination using the capsule endoscope 10 effectively in a short time.

When the control mode is switched from the water surface mode to the in-the-water mode in the same manner as in the first modified example, it is possible to acquire the control condition at the first time and guide the capsule endoscope 10 according to the control condition thereafter in the same manner as in the second embodiment.

Further, when the magnetic field generation unit is formed by using a plurality of electromagnets 31a illustrated in FIG. 14 in the same manner as in the second modified example, it is possible to perform the same control as that in the second embodiment. The control condition in this case is the strength of the current flowing through each electromagnet 31a when the capsule endoscope 10 floats up from the water bottom WB and remains still in the water.

In the second embodiment described above, the control condition is stored in the storage unit 27 and the guidance controller 261 sets a switching condition based on the control condition and performs control of the magnetic field generation unit 25. However, it is also possible to store a switching condition that is set based on the control condition into the guidance controller 261 and perform control of the magnetic field generation unit 25 based on the switching condition.

### (Third Embodiment)

Next, a third embodiment of the present invention will be described. A configuration of a capsule medical device guidance system according to the third embodiment of the present invention is the same as that of the first embodiment illustrated in FIGS. 1 to 3, and a control operation performed by the control unit 26 is different from that of the first embodiment.

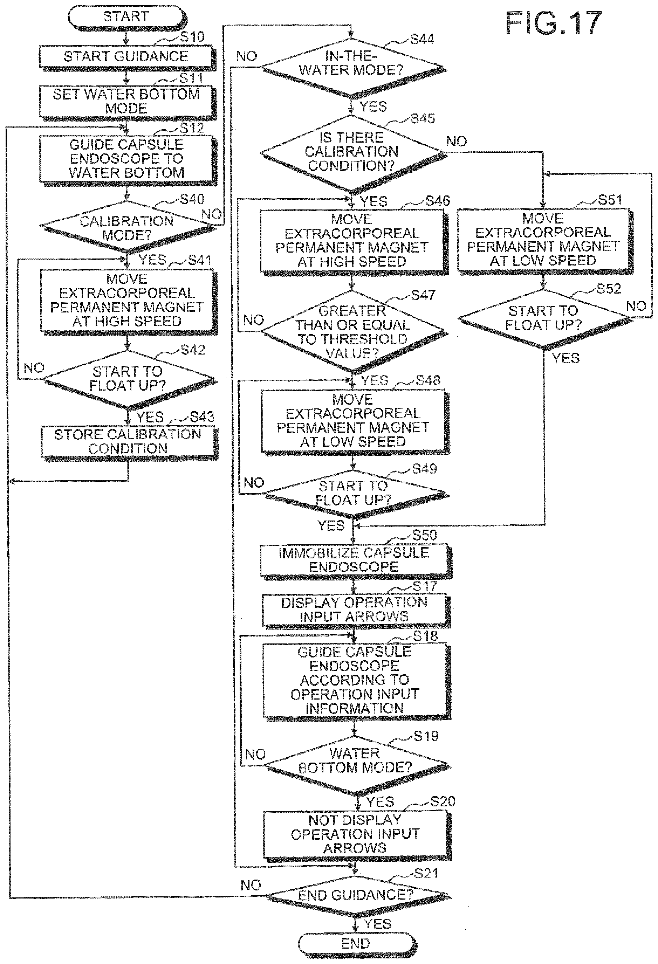

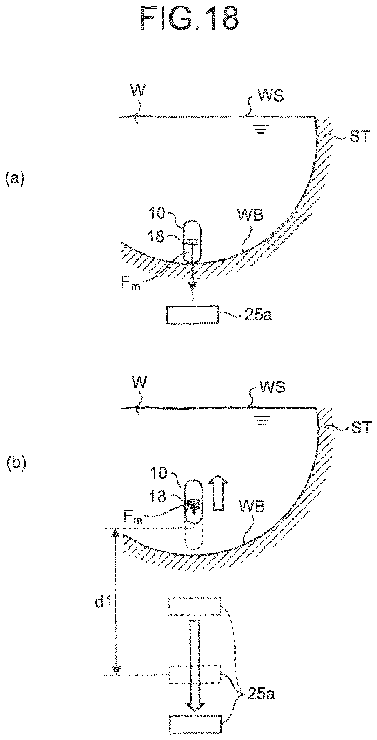

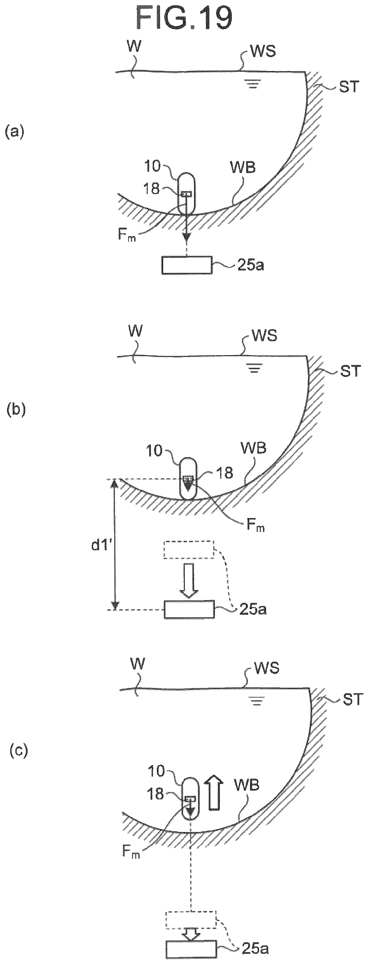

FIG. 17 is a flowchart illustrating an operation of the capsule medical device guidance system (hereinafter also referred to as simply a system) 1 according to the third embodiment of the present invention. FIGS. 18 and 19 are schematic diagrams for explaining a guidance method of the capsule endoscope 10 of the system 1. Steps S10 to S12 illustrated in FIG. 17 are the same as those in the first embodiment.

In step S40 following step S12, the control unit 26 determines whether or not instruction information for switching the control mode to a calibration mode which is a fourth control mode is inputted from the operation input unit 24.

When the instruction information for switching the control mode to the calibration mode is inputted (step S40: Yes), the guidance controller 261 moves the extracorporeal permanent magnet 25a vertically downward at a high speed (step S41) and reduces the magnetic attracting force Fₘ applied to the permanent magnet 18 as illustrated in (a) and (b) of FIG. 18. In this calibration mode, it is not necessary to immobilize the capsule endoscope 10 in the water, so that the moving speed of the extracorporeal permanent magnet 25a at this time may be higher than that in step S14 of FIG. 9 in the first embodiment. When the moving speed of the extracorporeal permanent magnet 25a is set to high, the magnetic field generated near the capsule endoscope 10 can be changed in a short time. That is to say, it is possible to increase the amount of change in the magnetic field per unit time.

In the next step S42, the determination unit 262 determines whether or not the capsule endoscope 10 detaches from the water bottom WB and starts to float up. The method for determining the above is the same as that in step S15 of FIG. 9 described in the first embodiment. When the capsule endoscope 10 has not yet started to float up (step S42: No), the guidance controller 261 continues to move the extracorporeal permanent magnet 25a (step S41).

On the other hand, when the capsule endoscope 10 detaches from the water bottom WB and starts to float up (step S42: Yes), the guidance controller 261 acquires a relative position of the extracorporeal permanent magnet 25a with respect to the capsule endoscope 10 of when the capsule endoscope 10 detaches from the water bottom WB and starts to float up, that is, a distance d1 between the capsule endoscope 10 and the extracorporeal permanent magnet 25a illustrated in (b) of FIG. 18, as a control condition, determines a value d1' close to the distance d1 (d1' < d1) as a threshold value, and stores the threshold value d1' in the storage unit 27 as a calibration condition (a switching condition) (step S43). Thereafter, the operation of the system 1 returns to step S12.

When the instruction information for switching the control mode to the calibration mode is not inputted in step S40 (step S40: No), the control unit 26 determines whether or not instruction information for switching the control mode to the in-the-water mode is inputted from the operation input unit 24 (step S44). When the instruction information for switching the control mode to the in-the-water mode is not inputted (step S44: No), the operation of the system 1 proceeds to step S21.

On the other hand, when the instruction information for switching the control mode to the in-the-water mode is inputted (step S44: Yes), the control unit 26 determines whether or not the calibration condition is stored in the storage unit 27 (step S45).

When the calibration condition is stored in the storage unit 27 (step S45: Yes), the guidance controller 261 moves the extracorporeal permanent magnet 25a vertically downward at a high speed (step S46) as a fifth control mode and reduces the magnetic attracting force Fₘ applied to the permanent magnet 18 as illustrated in (a) and (b) of FIG. 19. The moving speed of the extracorporeal permanent magnet 25a at this time may be higher than that in step S14 of FIG. 9 in the first embodiment.

In the next step S47, the determination unit 262 compares the relative position of the extracorporeal permanent magnet 25a with respect to the capsule endoscope 10 with the calibration condition and determines whether or not to proceed to the next step S48. In detail, it is determined whether or not a current distance between the capsule endoscope 10 and the extracorporeal permanent magnet 25a is greater than or equal to the threshold value d1' that is set as the calibration condition. When the distance is smaller than the threshold value d1' (step S47: No), the guidance controller 261 continues to move the extracorporeal permanent magnet 25a (step S46).

In step S47, control is performed based on the distance between the capsule endoscope 10 and the extracorporeal permanent magnet 25a. However, the control may be performed based on the position of the extracorporeal permanent magnet 25a by setting the position of the extracorporeal permanent magnet 25a corresponding to the threshold value d1' as the calibration condition in advance.

On the other hand, when the distance is greater than or equal to the threshold value d1' (step S47: Yes), the guidance controller 261 reduces the moving speed of the extracorporeal permanent magnet 25a as a sixth control mode and moves the extracorporeal permanent magnet 25a vertically downward at a low speed (step S48). Thereby, as illustrated in (c) of FIG. 19, the guidance controller 261 gradually reduces the magnetic attracting force Fₘ applied to the permanent magnet 18. Preferably, the moving speed should be similar to that in step S14 of FIG. 9 in the first embodiment. When the moving speed of the extracorporeal permanent magnet 25a is set to low, it is possible to reduce the amount of change per unit time in the magnetic field generated near the capsule endoscope 10.

In the next step S49, the determination unit 262 determines whether or not the capsule endoscope 10 detaches from the water bottom WB and starts to float up. The method for determining the above is the same as that in the first embodiment (see step S15 in FIG. 9). When the capsule endoscope 10 has not yet started to float up (step S49: No), the guidance controller 261 continues to move the extracorporeal permanent magnet 25a (step S48).

On the other hand, when the capsule endoscope 10 detaches from the water bottom WB and starts to float up (step S49: Yes), the control mode of the guidance controller 261 switches to the floating mode, and the guidance controller 261 stops the movement of the extracorporeal permanent magnet 25a and immobilizes the capsule endoscope 10 in the water (step S50). The subsequent steps S17 to S21 are the same as those in the first embodiment (see FIG. 9).

When the calibration condition is not stored in the storage unit 27 in step S45 (step S45: No), the guidance controller 261 moves the extracorporeal permanent magnet 25a vertically downward at a low speed (step S51) and gradually reduces the magnetic attracting force Fₘ applied to the permanent magnet 18. The moving speed of the extracorporeal permanent magnet 25a at this time is preferred to be substantially the same as that in step S14 of FIG. 9 in the first embodiment.

In the next step S52, the determination unit 262 determines whether or not the capsule endoscope 10 detaches from the water bottom WB and starts to float up. The method for determining the above is the same as that in step S15 of FIG. 9 described in the first embodiment. When the capsule endoscope 10 has not yet started to float up (step S52: No), the guidance controller 261 continues to move the extracorporeal permanent magnet 25a (step S51).

On the other hand, when the capsule endoscope 10 detaches from the water bottom WB and starts to float up (step S52: Yes), the operation of the system 1 proceeds to step S50.

As described above, according to the third embodiment, when acquiring a calibration condition according each capsule endoscope 10 and switching the control mode from the water bottom mode to the in-the-water mode, the extracorporeal permanent magnet 25a is moved close to the calibration condition at a high speed and then the extracorporeal permanent magnet 25a is moved at a low speed while determining whether or not the capsule endoscope 10 detaches from the water bottom WB and starts to float up, so that it is possible to quickly and stably perform the control of the magnetic field for causing the capsule endoscope 10 to float up from the water bottom WB and remain still in the water.

When the control mode is switched from the water surface mode to the in-the-water mode in the same manner as in the first modified example, it is possible to acquire a calibration condition and guide the capsule endoscope 10 by using the calibration condition in the same manner as in the third embodiment.

When forming a magnetic field generation unit by using a plurality of electromagnets 31a illustrated in FIG. 14 in the same manner as in the second modified example, it is possible to perform the same control as that of the third embodiment. In this case, the capsule endoscope 10 is floated up by generally weakening the current flown in each electromagnet 31a in steps S41, S46, S48, and S51 illustrated in FIG. 17. The calibration condition in this case is the strength of the current flowing through each electromagnet 31a when the capsule endoscope 10 floats up from the water bottom WB and remains still in the water or a value close to the strength. In step S47, it is determined whether or not the strength of the current at that time is smaller than or equal to the value that is set as the calibration condition. Further, in this case, the amount of change per unit time in the current flown through each electromagnet 31a is increased in steps S41, S46, and S51 illustrated in FIG. 17, and the amount of change per unit time in the current is decreased in step S48. Thereby it possible to obtain the same effect as when the moving speed of the extracorporeal permanent magnet 25a is changed.

In the third embodiment described above, the value d1' close to the distance d1 between the capsule endoscope 10 and the extracorporeal permanent magnet 25a is stored in the storage unit 27 as the calibration condition. However, when an inclination of the capsule endoscope 10 or the extracorporeal permanent magnet 25a changes, a relationship between the distance between the capsule endoscope 10 and the extracorporeal permanent magnet 25a and the magnetic attracting force generated for the capsule endoscope 10 changes, and accordingly also the calibration condition changes.

Therefore, to avoid such a situation, the inclination of the capsule endoscope 10 or the extracorporeal permanent magnet 25a may be stored in the storage unit 27 along with the value d1'. Alternatively, the magnetic attracting force applied to the capsule endoscope 10 may be stored, or values required to calculate the magnetic attracting force applied to the capsule endoscope 10, specifically, the direction, the strength, and the gradient of the magnetic field generated near the capsule endoscope 10 may be stored. Thereby, even when the inclination of the capsule endoscope 10 or the extracorporeal permanent magnet 25a changes, it is possible to convert the value d1', which is the calibration condition, to an optimal value.

### (Fourth Embodiment)

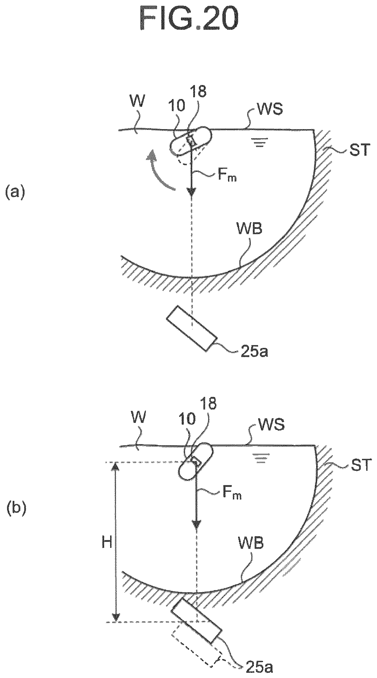

Next, a fourth embodiment of the present invention will be described. A configuration of a capsule medical device guidance system according to the fourth embodiment of the present invention is the same as that of the first embodiment illustrated in FIGS. 1 to 3, and a control operation performed by the control unit 26 is different from that of the first embodiment. FIG. 20 is a schematic diagram for explaining a guidance method of a capsule endoscope 10 of the capsule medical device guidance system according to the fourth embodiment.

In the case of changing the posture of the capsule endoscope 10 in a state in which the capsule endoscope 10 is in contact with the water surface WS or the water bottom WB, when guiding the capsule endoscope 10 under a condition in which the magnetic attracting force Fₘ, the buoyant force, and the gravitational force, which are applied to the capsule endoscope 10, are balanced, if the magnetic attracting force Fₘ is too strong or too weak, it may be difficult to control the posture of the capsule endoscope 10 as intended by a user. When maintaining the state in which the capsule endoscope 10 is in contact with the water surface WS, the above tendency becomes further strong due to the effect of surface tension. For example, as illustrated in (a) of FIG. 20, when causing the capsule endoscope 10 to come into contact with the water surface WS, the magnetic attracting force Fₘ is relatively insufficient due to the effect of surface tension, so that the capsule endoscope 10 falls down in a direction in parallel with the water surface WS.

Therefore, in the fourth embodiment, after starting the guidance of the capsule endoscope 10, first, a distance H between the capsule endoscope 10 and the extracorporeal permanent magnet 25a when the capsule endoscope 10 starts to float up is acquired by performing a calibration. Then, the control unit 26 calculates an acting force applied to the capsule endoscope 10 at this time at the distance H based on magnetic characteristics, specifically a magnetic moment of the capsule endoscope 10 and a magnetic field distribution generated by the extracorporeal permanent magnet 25a.

Here, in a case in which the specific weight of the capsule endoscope 10 is smaller than 1, when maintaining a state in which the capsule endoscope 10 is in contact with the water surface WS, the position of the extracorporeal permanent magnet 25a is controlled so that a downward magnetic attracting force applied to the capsule endoscope 10 becomes small with reference to the calculated acting force. On the other hand, when maintaining a state in which the capsule endoscope 10 is in contact with the water bottom WB, the position of the extracorporeal permanent magnet 25a is controlled so that a downward force applied to the capsule endoscope 10 becomes large with reference to the calculated acting force.

In a case in which the specific weight of the capsule endoscope 10 is greater than 1, when maintaining a state in which the capsule endoscope 10 is in contact with the water surface WS, the position of the extracorporeal permanent magnet 25a is controlled so that an upward magnetic attracting force applied to the capsule endoscope 10 becomes large with reference to the calculated acting force. On the other hand, when maintaining a state in which the capsule endoscope 10 is in contact with the water bottom WB, the position of the extracorporeal permanent magnet 25a is controlled so that a downward force applied to the capsule endoscope 10 becomes small with reference to the calculated acting force.

The control unit 26 performs control of the magnetic field for changing the posture of the capsule endoscope 10 while maintaining the states as described above. Thereby, as illustrated in (b) of FIG. 20, an appropriate magnetic attracting force Fₘ is constantly applied to the permanent magnet 18 built in the capsule endoscope 10. Therefore, the posture of the capsule endoscope 10 can be maintained as intended by a user.

The first to the fourth embodiments and the modified examples described above are merely examples for implementing the present invention and the present invention is not limited to these embodiments and modified examples. Various inventions can be formed from the present invention by appropriately combining a plurality of components disclosed in the first to the fourth embodiments and the modified examples. It is clear from the above description that the present invention can be variously modified according to specifications and the like, and further other various embodiments can be made within the scope of the present invention.

### Reference Signs List

- 1, 2: CAPSULE MEDICAL DEVICE GUIDANCE SYSTEM
- 10: CAPSULE ENDOSCOPE
- 11: IMAGING UNIT
- 12: ILLUMINATION UNIT
- 13: OPTICAL SYSTEM
- 14: IMAGE SENSOR
- 15: CONTROL UNIT
- 16: WIRELESS COMMUNICATION UNIT
- 16a: ANTENNA
- 17: POWER SUPPLY UNIT
- 18: PERMANENT MAGNET
- 19: POSITION DETECTION MAGNETIC FIELD GENERATION UNIT
- 19a: MARKER COIL
- 19b: CAPACITOR
- 20,: 30 GUIDANCE DEVICE
- 20a: BED
- 21: RECEIVING UNIT
- 21a: RECEIVING ANTENNA
- 22: POSITION AND POSTURE DETECTION UNIT
- 22a: SENSING COIL
- 23: DISPLAY UNIT
- 24: OPERATION INPUT UNIT
- 25, 31: MAGNETIC FIELD GENERATION UNIT
- 25a: EXTRACORPOREAL PERMANENT MAGNET
- 25b: PLANE POSITION CHANGE UNIT
- 25c: VERTICAL POSITION CHANGE UNIT
- 25d: ELEVATION ANGLE CHANGE UNIT
- 25e: TURNING ANGLE CHANGE UNIT
- 26: CONTROL UNIT
- 27: STORAGE UNIT
- 31a: ELECTROMAGNET
- 31b: POWER SUPPLY UNIT
- 31c: CURRENT CONTROLLER
- 100: CAPSULE-SHAPED CASING
- 101: TUBULAR CASING
- 102, 103: DOME-SHAPED CASING

## Claims

1. A guidance device for introducing a capsule medical device having therein a magnet, into a subject in which a liquid is introduced, and guiding the capsule medical device in the liquid by using a magnetic field, the guidance device comprising:
a magnetic field generation unit configured to generate a magnetic field to be applied to the magnet; and
a guidance controller configured to control the magnetic field generated by the magnetic field generation unit, wherein
the guidance controller is configured to switch between:
a first control mode that causes the magnetic field generation unit to generate a magnetic field that guides the capsule medical device in a direction in which the capsule medical device moves and comes into contact with an upper or a lower boundary surface of the liquid;
a second control mode that continuously changes the magnetic field generated by the magnetic field generation unit so that the capsule medical device shifts from a state in which the capsule medical device is in contact with the boundary surface to a state in which the capsule medical device is not in contact with the boundary surface; and
a third control mode that controls the magnetic field generated by the magnetic field generation unit so that the capsule medical device is held in the liquid.

2. The guidance device according to claim 1, further comprising a determination unit configured to determine whether or not the capsule medical device is in contact with the boundary surface, wherein
the guidance controller is configured to switch to the third control mode based on a determination result where the determination unit determines that the capsule medical device is not in contact with the boundary surface in the second control mode.

3. The guidance device according to claim 2, wherein
based on a control condition for the magnetic field generation unit in switching from the second control mode to the third control mode based on the determination result by the determination unit, the guidance controller is configured to determine a switching condition for subsequent switching from the second control mode to the third control mode.

4. The guidance device according to claim 1, further comprising a determination unit configured to determine whether or not the capsule medical device is in contact with the boundary surface, wherein
the guidance controller is configured to:
further perform a fourth control mode that continuously changes the magnetic field generated by the magnetic field generation unit so that the capsule medical device shifts from a state in which the capsule medical device is in contact with the boundary surface to a state in which the capsule medical device is not in contact with the boundary surface, and acquires a control condition for the magnetic field generation unit at a time when the determination unit determines that the capsule medical device shifts from a state in which the capsule medical device is in contact with the boundary surface to a state in which the capsule medical device is not in contact with the boundary surface; and
determine, in the second control mode, a switching condition in switching from the second control mode to the third control mode, based on the control condition acquired by the fourth control mode.

5. The guidance device according to claim 3 or 4, wherein
in switching from the second control mode to the third control mode, the guidance controller is configured to control the magnetic field generation unit such that an amount of change in a magnetic field per unit time when continuously changing a magnetic field generated by the magnetic field generation unit is greater when switching according to the switching condition than when switching based on the determination result by the determination unit.

6. The guidance device according to claim 4, wherein
the guidance controller is configured to continuously change the magnetic field generated by the magnetic field generation unit such that an amount of change in the magnetic field per unit time in the fourth control mode is greater than an amount of change in the magnetic field per unit time in the second control mode when switching to the third control mode based on the determination result by the determination unit.

7. The guidance device according to claim 3 or 4, wherein
the second control mode includes:
a fifth control mode that continuously changes the magnetic field generated by the magnetic field generation unit in a direction in which the capsule medical device shifts from a state in which the capsule medical device is in contact with the boundary surface to a state in which the capsule medical device is not in contact with the boundary surface; and
a sixth control mode that continuously changes the magnetic field generated by the magnetic field generation unit so that the capsule medical device shifts to the state in which the capsule medical device is not in contact with the boundary surface based on the determination result by the determination unit after the fifth control mode, wherein
the guidance controller is configured to control the magnetic field generation unit such that an amount of change in the magnetic field per unit time in the fifth control mode is greater than an amount of change in the magnetic field per unit time in the sixth control mode.

8. The guidance device according to claim 2 or 4, further comprising a position detection unit configured to detect a position of the capsule medical device in the subject and output position information, wherein
the determination unit is configured to determine whether or not the capsule medical device is in contact with the boundary surface, based on the position information.

9. The guidance device according to claim 2 or 4, wherein
the capsule medical device comprises an imaging unit for imaging an inside of the subject to generate image data, and a transmitting unit for wirelessly transmitting the image data, and
the determination unit is configured to determine whether or not the capsule medical device is in contact with the boundary surface based on the image data wirelessly transmitted from the capsule medical device.

10. The guidance device according to claim 1, further comprising an operation input unit configured to input instruction information for changing at least one of position and posture of the capsule medical device into the guidance controller according to an operation performed from outside, wherein
the guidance controller is configured to determine whether or not to perform control based on the instruction information according to a control mode being performed.

11. The guidance device according to claim 10, wherein
the guidance controller is configured to determine not to perform the control based on the instruction information even when the instruction information is inputted from the operation input unit while the second control mode is being performed.

12. The guidance device according to claim 4, further comprising an operation input unit configured to input instruction information for changing at least one of position and posture of the capsule medical device into the guidance controller according to an operation performed from outside, wherein
the guidance controller is configured to determine not to perform control based on the instruction information even when the instruction information is inputted from the operation input unit while the fourth control mode is being performed.

13. The guidance device according to claim 7, further comprising an operation input unit configured to input instruction information for changing at least one of position and posture of the capsule medical device into the guidance controller according to an operation performed from outside, wherein
the guidance controller is configured to determine not to perform control based on the instruction information even when the instruction information is inputted from the operation input unit while the sixth control mode is being performed.

14. The guidance device according to any one of claims 10, 12, and 13, further comprising a display unit configured to display a sign showing a state whether or not to perform control based on the instruction information, wherein
the display unit is configured to switch the state of the sign according to a control mode being performed by the guidance controller.

15. A capsule medical device guidance system comprising:
the guidance device according to claim 1; and
the capsule medical device.
